# EUROPÄISCHE PATENTSCHRIFT

(11) **EP 2 242 734 B1**
(45) Veröffentlichungstag und Bekanntmachung des Hinweises auf die Patenterteilung: **06.09.2017**
(21) Anmeldenummer: 09703676.8
(22) Anmeldetag: 15.01.2009
(51) Int. Cl.: C07C 5/03, C07C 9/22, C07C 41/09, C07C 43/04, C09K 5/06

(54) **VERFAHREN ZUR HERSTELLUNG EINES LATENTWÄRMESPEICHERMATERIALS**
METHOD FOR PRODUCING A LATENT HEAT STORAGE MATERIAL
PROCÉDÉ DE FABRICATION D'UN MATÉRIAU A CHANGEMENT DE PHASE

(30) Priorität: 23.01.2008 DE 102008005721
(43) Veröffentlichungstag der Anmeldung: 27.10.2010
(73) Patentinhaber: SASOL Germany GmbH, 20537 Hamburg (DE)
(72) Erfinder: ZIEHE, Holger, 25524 Itzehoe (DE); WEITZE, Achim, 25541 Brunsbüttel (DE); GROSS, Thoralf, 25541 Brunsbüttel (DE)
(74) Vertreter: Müller Schupfner & Partner Patent- und Rechtsanwaltspartnerschaft mbB
(86) Internationale Anmeldenummer: PCT/DE2009/000036
(87) Internationale Veröffentlichungsnummer: WO 2009/092349

(56) Entgegenhaltungen:
- WO-A-2004/092299
- WO-A-2006/097222
- WO-A-2007/058003
- WO-A1-2007/107171
- DE-A1-102004 056 786
- US-A- 5 650 546
- EUGEN MÜLLER: "Houben-Weyl's Methoden der Organischen Chemie, Band V/1a, Teil 1, Kohlenwasserstoffe" 1970, GEORG THIEME VERLAG , STUTTGART , XP002531530 Seite 8
- CHO J-S ET AL: "Microencapsulation of octadecane as a phase-change material by interfacial polymerization in an emulsion system", COLLOID & POLYMER SCIENCE, SPRINGER VERLAG, HEIDELBERG, DE, vol. 280, 1 January 2002 (2002-01-01), pages 260-266, XP002462568, ISSN: 0303-402X, DOI: 10.1007/S00396-001-0603-X
- ALVARADO J L ET AL: "Characterization of supercooling suppression of microencapsulated phase change material by using DSC", JOURNAL OF THERMAL ANALYSIS AND CALORIMETRY, KLUWER ACADEMIC PUBLISHERS, DORDRECHT, NL, vol. 86, no. 2, 6 October 2006 (2006-10-06), pages 505-509, XP019436340, ISSN: 1572-8943, DOI: 10.1007/S10973-005-7430-0
- He Bo ET AL: "Tetradecane and hexadecane binary mixtures as phase change materials (PCMs) for cool storage in district cooling systems", ENERGY, vol. 24, no. 12, 1 January 1999 (1999-01-01), pages 1015-1028, XP055232681, GB ISSN: 0360-5442, DOI: 10.1016/S0360-5442(99)00055-9
- M W Babich ET AL: "The search for novel energy storage materials using differential scanning caldrimetry", Thermochimica Acta Elsevier Science Publishers B.V.. Amsterdam, 1 January 1992 (1992-01-01), pages 83-88, XP055264092, Retrieved from the Internet: URL:http://ac.els-cdn.com/0040603192802796 /1-s2.0-0040603192802796-main.pdf?_tid=5e0 007ba-ffc1-11e5-a454-00000aab0f6b&acdnat=1 460364347_7f8bb6d1562854a2249c9a8f38ab6271
- SYUKRI HIMRAN ET AL: "Characterization of Alkanes and Paraffin Waxes for Application as Phase Change Energy Storage Medium", ENERGY SOURCES, vol. 16, no. 1, 1994, pages 117-128,

## Beschreibung

Die Erfindung betrifft ein Verfahren zur Herstellung von Latentwärmespeichermaterial aus linearen Alkoholen.

Phasentransfer-Materialien (PCMs - Phase Change Materials) können durch Schmelzen bzw. Erstarren innerhalb eines definierten Temperaturbereiches Wärme freisetzen bzw. absorbieren bzw. speichern und wirken somit als Latentwärmespeichermaterial. Dieses Prinzip der Wärmespeicherung kann man sich zum Beispiel in der Wandisolierung von, Gebäuden zunutze machen. Derartige Latentwärmespeichermaterialien werden z.B. in Form von Mikrokapseln in den Wandputz oder in Gipskartonplatten eingebracht und verflüssigen sich tagsüber während starker Wärmeeinwirkung. Die absorbierte Wärme wird in der Wand gespeichert und hält den Innenraum kühl. Nach Abkühlen in den Abendstunden und in der Nacht erstarren die flüssigen Speicher und geben die Kristallisationswärme an die Umgebung ab. Der Innenraum wird dabei erwärmt. Als Latentwärmespeichermaterialien werden überwiegend Paraffine und Paraffinmischungen eingesetzt (siehe Bo et al., Energy, 1999, 1015-1028, Babich et al., Thermochimica, 1992, 83-88 and Himran et al., Energy Sources, 1994, Vol. 16, 117-128). Kommerziell erhältliche Paraffinmischungen für PCM-Anwendungen sind beispielsweise Rubitherm® 27 und Rubitherm® 31. Die Hauptkomponente obiger Rubitherm®-Mischungen ist ein C₁₈ Paraffin mit einem Gehalt von nur 59 bzw. 39 Massen%. Diese Paraffinmischungen bestehen aus gerad- und ungeradzahligen linearen Paraffinen im Kettenlängenbereich von C₁₇ bis C₂₁, bzw. C₁₇ bis ca. C₃₀, weisen aber einen Anteil an linearen Ketten von 98,0 bzw. 95,6 Massen% auf.

Paraffine sind auch durch Hydrierung von kommerziell erhältlichen alpha-Olefinen herstellbar. Diese weisen im Bereich C₁₆ bis C₁₈ allerdings nur Linearitäten von ca. 90 bis kleiner 95 Massen% auf und haben den Nachteil, dass aufgrund der verzweigten Nebenprodukte deren Schmelzenthalpie im Vergleich deutlich niedriger ist als die hochlinearer Paraffine.

Es wurde gefunden, dass Mischungen aus geradzahligen und ungeradzahligen Paraffinen, solchen mit unterschiedlicher Kettenlängen und/oder höheren verzweigten Anteilen den Nachteil haben, dass diese breite oder mehrere unterschiedliche Schmelzpeaks aufweisen, wobei wenn diese hinsichtlich der Temperatur zu weit auseinander liegen, i.d.R. nur ein Teil der möglichen Schmelzenthalpie tatsächlich nutzbar ist.

Aufgabe der vorliegenden Erfindung ist es ein Verfahren zur Herstellung hochlinearer Paraffine, mit definierter Kettenlänge für die Verwendung als
Latentwärmespeichermaterial bereitzustellen. Hierdurch werden folgende Vorteile erreicht: Zum einen ist der Schmelzbereich hochreiner Paraffine im Vergleich zu Paraffingemischen deutlich enger und somit kann bereits bei geringen Temperaturdifferenzen die volle Speicherkapazität genutzt werden. Zum anderen ist die Schmelzenthalpie der Reinsubstanzen deutlich höher als die der Mischungen.

Die Erfindung ist durch den Gegenstand des Anspruchs 1 definiert. Bevorzugte Ausführungsformen sind Gegenstand der Unteransprüche oder nachfolgend beschrieben.

Reinsubstanzen, insbesondere lineare Paraffine definierter Kettenlängen, weisen dabei höhere Schmelzwärmen und schmalere Schmelzbereiche auf als verzweigte Paraffine oder Paraffinmischungen. Die Schmelztemperatur des PCM ist über die Wahl der Struktur und Kettenlänge des Paraffins über einen großen Temperaturbereich einstellbar.

Die Paraffine erfüllen vorzugsweise unabhängig voneinander folgende Spezifikation:
a) sie weisen zu größer 95 Massen%, insbesondere zu größer 98 Massen%, geradzahlige Kettenlängen auf,
b) sie weisen zu größer 95 Massen%, insbesondere zu größer 97 Massen%, ausschließlich eine bestimmte C-Zahl auf,
c) sie sind zu größer 95 Massen% linear.

Die Alkohole zur Herstellung der Paraffine sind so aufgereinigt, dass diese obige Grenzwerte für die Paraffine erfüllen.

Das Latentwärmespeichermaterial ist durch Dehydratisierung von linearen Fettalkoholen, zu Olefinen zugänglich, wobei diese anschließend zu Paraffinen hydriert werden. Fettalkohole im Sinne dieser Erfindung sind Alkohole mit C-Zahlen größer gleich 6 und vorzugsweise endständigen Hydroxy-Gruppen. Besonders geeignete Ausgangsmaterialien sind im Falle der Paraffine Cetylalkohol oder Stearylalkohol.

Es wurde somit überraschend gefunden, dass sich als lineare Paraffine für PCM-Anwendungen Paraffine eignen, die durch Dehydratisierung linearer Alkohole zu linearen Olefinen und anschließender Hydrierung herstellen lassen. Die eingesetzten linearen Alkohole sind als Einzelschnitte leicht verfügbar und basieren auf nachwachsenden pflanzlichen Rohstoffen, wie z. B. Palmöl, Palmkernöl, KokusöI, Rapsöl oder anderen pflanzlichen Ölen.

Aus natürlichen Rohstoffen zugängliche Alkohole zeichnen sich durch eine erhöhte Linearität von z.B > 98 Massen% aus. Die daraus gewonnenen Paraffine sind daher für die Anwendung in PCMs überraschend gut geeignet.

Die Verwendung von linearen Paraffinen als PCMs ist bekannt, ebenso wie die Herstellung von Paraffinen aus Fettalkoholen. Bislang sind aber keine Paraffine'für diese Anwendung als Latentwärmespeichermaterial beschrieben, die aus dehydratisierten Alkoholen hergestellt sind, insbesondere solchen, die aus nachwachsender Rohstoffen erhältlich sind.

Der Fachmann ist in der Vergangenheit stets davon ausgegangen, dass die Herstellung von Alkoholen aus Paraffinen ein Veredelungsschritt ist, bei dem der Alkohol einen höheren Wert als das Paraffin hat. Es wäre nun nicht zu erwarten, dass der umgekehrte Weg, d. h. die Herstellung eines Paraffins aus einem Alkohol wirtschaftlich sinnvoll ist. Es hat sich nun aber gezeigt, dass die Paraffine aus der Alkoholdehydratisierung zu besonders reinen Paraffinen führen und diese reinen Paraffine deutlich bessere Eigenschaften selbst im Vergleich zu nur geringfügig verunreinigten Paraffinen besitzen.

Für diese Spezialanwendung liegen die Preise der Paraffine oberhalb der Preise für Fettalkohole. Die benötigte Einsatzmenge an Latentwärmespeicher korreliert direkt mit der genutzten Schmelzwärme, d. h. das Substanzen, die eine 20 % höhere Schmelzwärme besitzen auch entsprechend weniger eingesetzt werden müssen, um den gleichen Effekt zu erzielen. Beispielsweise können Textilien gleicher Speicherkapazität mit geringerem Gewicht hergestellt und somit kann der Tragekomfort deutlich erhöht werden.

Das Latentwärmespeichermaterial wird vorzugsweise von einem Polymermaterial als Kapselwand zu Mikrokapseln mit mittleren Teilchengrößen im Bereich von 1 bis 200 µm oder zu Makrokapseln mit mittleren Teilchengrößen im Bereich von größer 200 µm bis 2 cm gekapselt. Geeignete Polymermaterialien sind z.B. Styrol-Divinylbenzol-Polymere, oder ungesättigte Polyester, Bevorzugte Wandmaterialien, da sehr alterungsstabil, sind insbesondere duroplastische Polymere. Geeignete duroplastische Polymermaterialien sind beispielsweise vernetzte Formaldehydharze, vernetzte Polyhamstoffe und vernetzte Polyurethane sowie vernetzte Methaerylsäurcesterpolymere.

Die Schmelztemperatur und die Schmelzwärme werden mittels DSC-Analytik bestimmt. Bei einer definierten Aufheiz- und Abkühlrate wird die Onset-Temperatur (Schmelztemperatur) und die Fläche unter der Kurve (Schmelzwärme) bestimmt. Die mittels DSC ermittelten Schmelztemperaturen und -wärmen der Paraffine und Paraffinmischungen sind jeweils im experimentellen Teil dargestellt.

Die Figuren zeigen:
Fig.1 C-Ketten Verteilung Paraffine Rubitherm® 27und Rubitherm® 31;

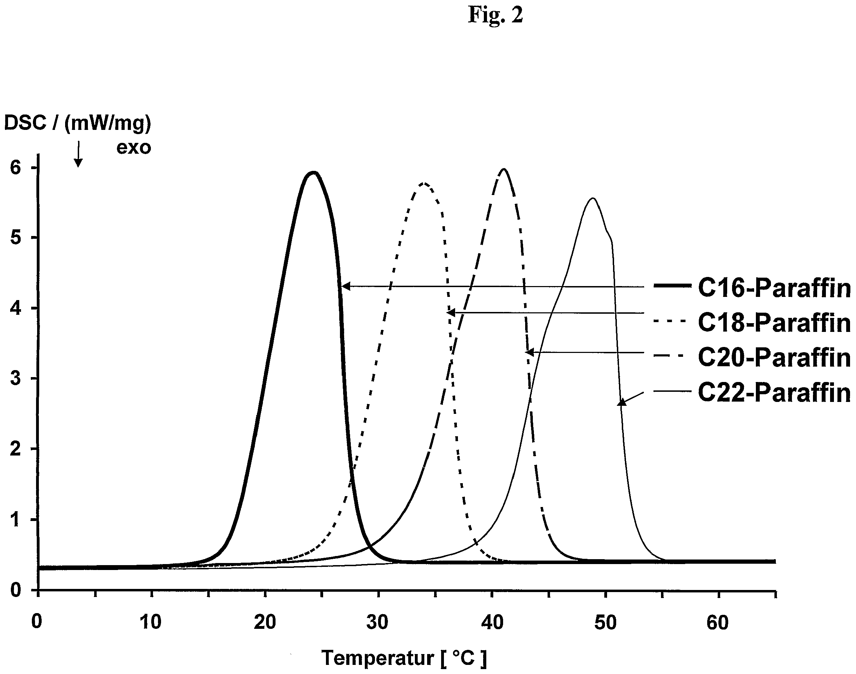

Fig. 2 DSC-Diagramme C16 bis C22 Paraffine (rein);

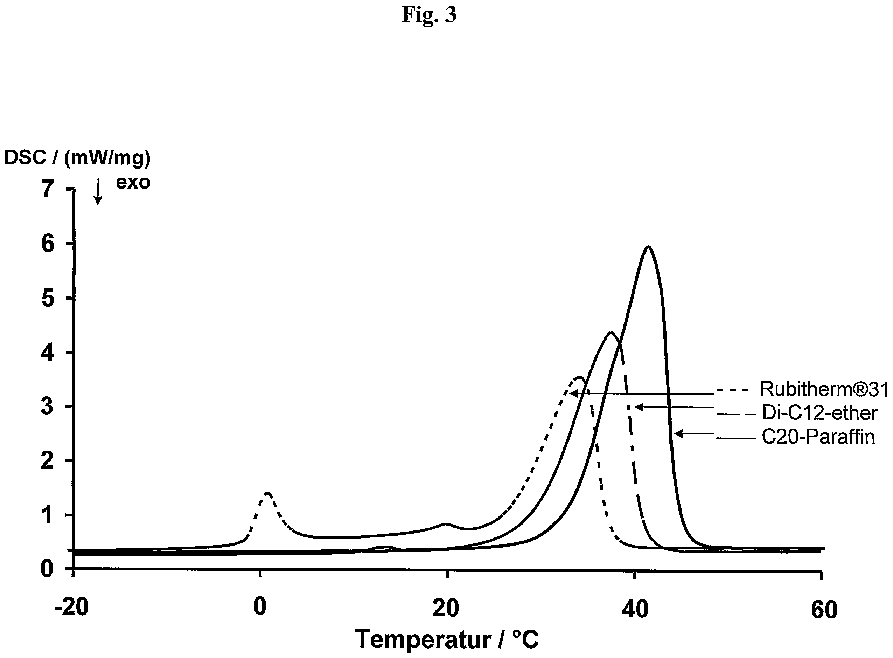

Fig.3 DSC-Diagramme Vergleich Rubitherm®31 (Vergleich)/ Di-C12Ether (nicht erfindungsgemäß) / C20-Paraffin;

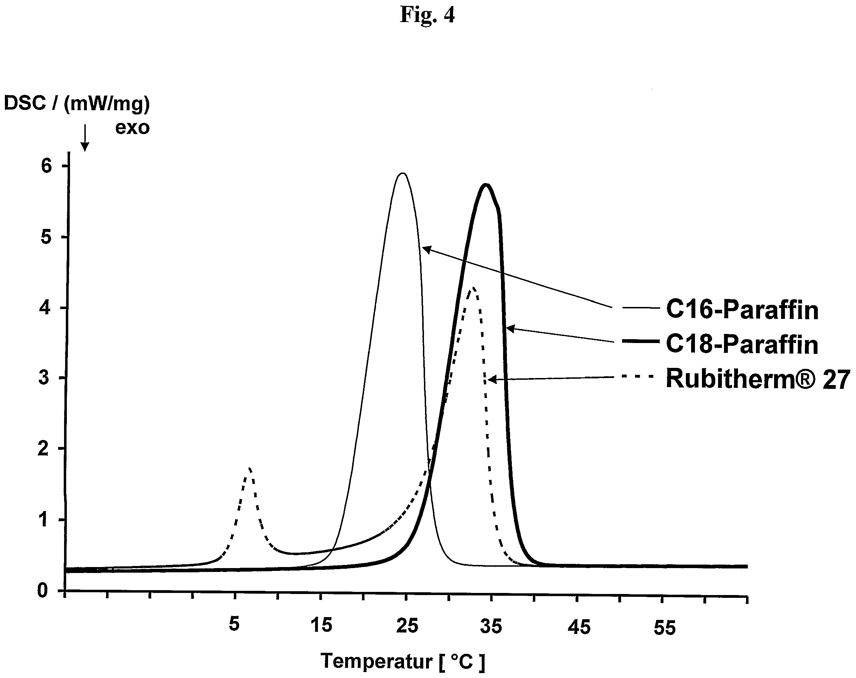

Fig. 4 DSC-Diagramme Vergleich Rubitherm® 27/C18-Paraffin / C16-Paraffin;

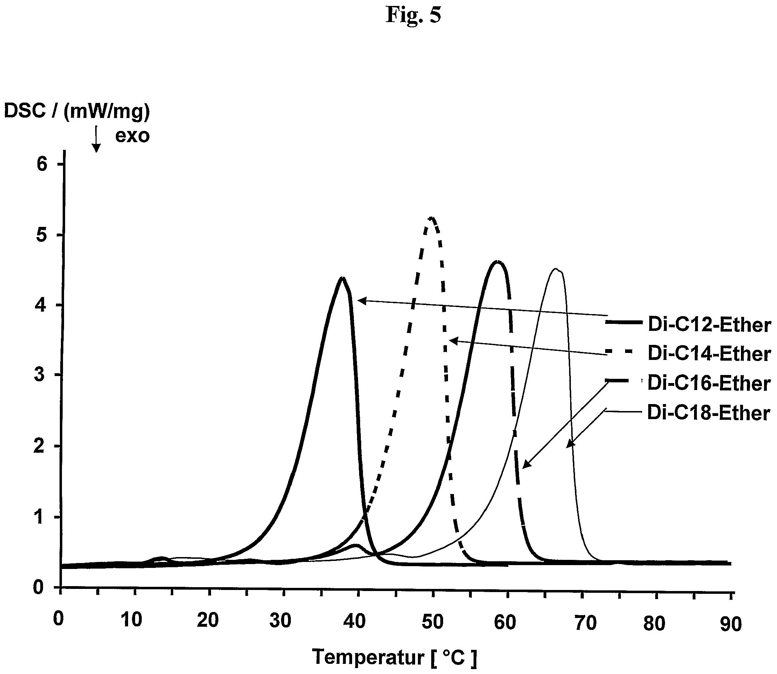

Fig.5 DSC Diagramme Di-C12/C14/C16/C18-Ether (nicht erfindungsgemäß) und

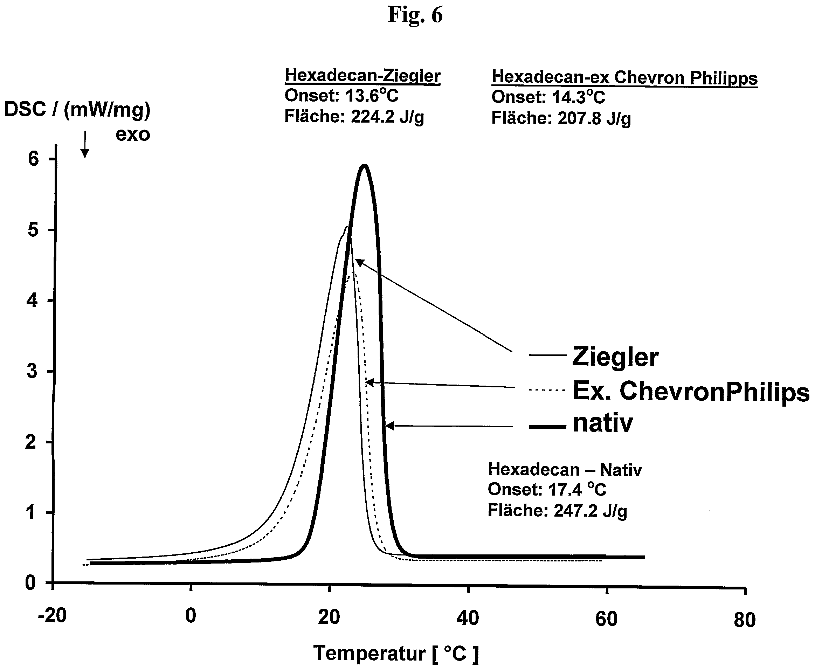

Fig.6 DSC-Diagramme Vergleich Hexadecan aus synthetischer / nativer Quelle.

### Experimenteller Teil

Die Auswertung der DSC-Analysen zur Bestimmung von Schmelzenthalpie [J/g] und Onset-Temperatur erfolgte nach der DIN 53765. Alle DSC-Kurven wurden mit dem Gerät DSC 204 F1 der Firma Netzsch bei Aufheiz- und Abkühlraten von 10 K/min gemessen.

### Vergleichsbeispiel:

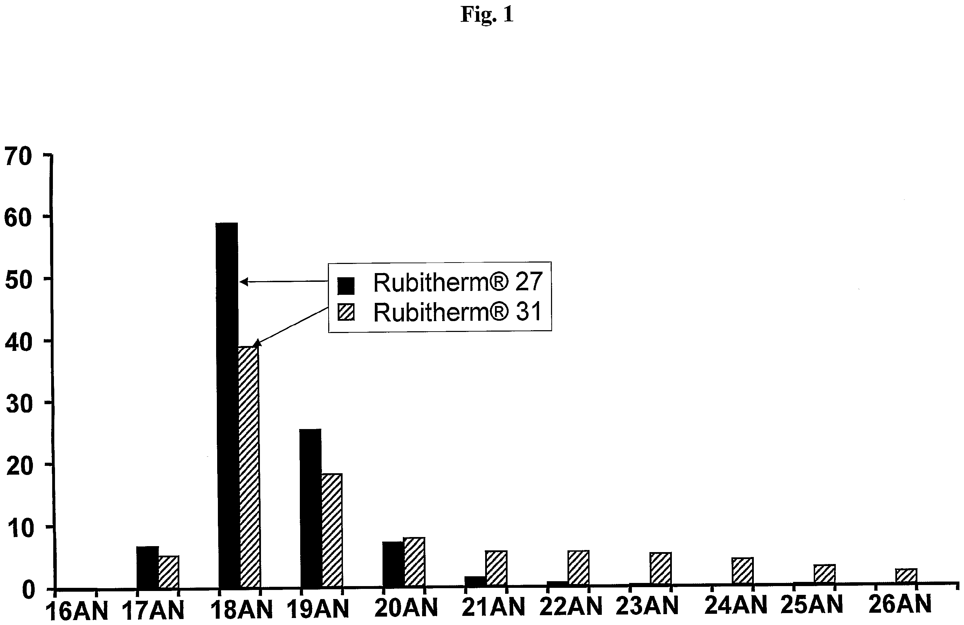

Kommerziell erhältliche PCMs sind Rubitherm® 27 und Rubitherm® 31: Rubitherm® 27 und Rubitherm® 31 haben die aus Figur 1 ersichtliche Zusammensetzung (per GC ermittelt) und zeigen im Weiteren die nachfolgende per DSC ermittelte Charakteristik.:

**Tabelle 1**

| Paraffin | Rubitherm® 27 | Rubitherm® 31 |
|---|---|---|
| n-Paraffin-Gehalt [%] | 98,0 | 95,6 |
| Onset 1 [°C] | 4 | - 2 |
| Onset 2 [°C] | 26 | 27 |
| Schmelzwärme 1 [J/g] | 22,0 | 17,9 |
| Schmelzwärme 2 [J/g] | 156,3 | 147,8 |

Exemplarisch für die Dehydratisierung von linearen Fettalkoholen wird nachfolgend die Dehydratisierung von Hexadecanol zu linearen Olefinen (Experiment 1) und die Hydrierung von Hexadecen (Experiment 2) beschrieben.

### Experiment 1: Dehydratisierung von Fettalkoholen zu linearen Olefinen

2474 g NACOL® 16-99 (Reinheit 99,5 %, basierend auf nachwachsenden Rohstoffen) wurden in einem 6-L-Kolben mit 500 g Al₂O₃ und 60 mL Xylol versetzt und bei bis zu 295 °C 4,5 Stunden am Wasserabscheider erhitzt. Dabei bildeten sich 180 mL Wasser. Das gebildete Hexadecen wurde im Vakuum destilliert. Erhalten wurde ein Gemisch aus alpha- und internen Olefinen.

### Experiment 2: Hydrierung von linearen Olefinen zu linearen Paraffinen

685 g des im Versuch 1 gewonnenen Hexadecens wurden nach bekanntem Verfahren über einem heterogenen Ni-haltigen Katalysator bei 20 bar H₂-Druck bei 98 °C über 7 Stunden hydriert und nach dem Abkühlen filtriert.

Fettalkohole mit Kettlängen von C₁₆ bis C₂₂ wurden gemäß Experiment 1 und 2 eingesetzt und folgende Paraffine wurden erhalten:

**Tabelle 2**

| Paraffin | Hexadecan | Octadecan | Eicosan | Docosan |
|---|---|---|---|---|
| n-Paraffin (Hauptkomponente) [%] | 99,6 | 98,8 | 93,2 | 97,4 |
| n-Paraffine (gesamt) [%] | 99,8 | 98,9 | 96,8 | 98,6 |
| iso-Paraffin [%] | 0,2 | 0,1 | 1,8 | 1,2 |
| Onset [°C] | 17,4 | 27,4 | 32,5 | 40,6 |
| Schmelzwärme [J/g] | 245,6 | 250,7 | 247,2 | 270,5 |

### Experiment 3:

Die Versuche 1 und 2 wurden wiederholt, jedoch wurde ein synthetischer Fettalkohol (Hexadecanol) aus dem Ziegler-Prozess mit einer Reinheit von 95,6 % als Einsatzalkohol verwendet.

### Experiment 4 (Vergleich):

Der Versuch 2 wurde wiederholt, jedoch wurde ein synthetisches Olefin (Hexadecen ex Chevron Phillips) mit einer Reinheit von 94,2 % als Einsatzolefin verwendet.

Ein Vergleich der Onset-Temperaturen und Schmelzwärmen für Paraffine unterschiedlicher Reinheiten aufgrund unterschiedlicher Herstellmethoden sind exemplarisch für das Hexadecan in folgender Tabelle zusammengestellt.

**Tabelle 3**

| Paraffin | Hexadecan | Hexadecan | Hexadecan |
|---|---|---|---|
| Versuchsnummer | 1 | 3 | 4 |
| Quelle | Nativer Alkohol | Synth. Alkohol | Synth. Olefin |
| n-C₁₆-Paraffin [%] | 99,6 | 91,8 | 92,3 |
| n-Paraffine (gesamt) [%] | 99,8 | 93,1 | 93,2 |
| Iso-Paraffine (gesamt) [%] | 0,2 | 6,3 | 6,2 |
| Onset [°C] | 17,4 | 13,6 | 14,3 |
| Schmelzwärme [J/g] | 245,6 | 224,2 | 207,8 |

### Experiment 5-7 (Vergleich):

Octadecan und Docosan wurden im Gewichts-Verhältnis 1:1, 2:1 und 3:1 gemischt und die DSC-Kurven wurden erneut vermessen.

**Tabelle 4**

| Paraffin-Mischung | C₁₈/C₂₂-Paraffin | C₁₈/C₂₂-Paraffin | C₁₈/C₂₂-Paraffin |
|---|---|---|---|
| [Gew.-Verhältnis] | 1:1 | 2:1 | 3:1 |
| Onset 1 [°C] | -1,6 | -1,2 | -0,5 |
| Onset 2 [°C] | 28,5 | 26,6 | 26,7 |
| Schmelzwärme 1 [J/g] | 17,67 | 21,64 | 19,93 |
| Schmelzwärme 2 [J/g] | 123,9 | 128,7 | 123,4 |

Exemplarisch für die partielle Dehydratisierung von linearen Fettalkoholen wird nachfolgend die Dehydratisierung von Dodecanol zu linearen Dialkylethern beschrieben.

### Experiment 8.11 (nicht erfindungsgemäß): Dehydratisierung von linearen Fettalkoholen zu Dialkylethern

10 kg/h NACOL® 12-99 (Reinheit 99,2 %, basierend auf nachwachsenden Rohstoffen) wurden nach bekanntem Verfahren in einem Festbettreaktor (∅ = 60 mm, 1 = 900 mm) bei 260 °C über Al₂O₃-Kugeln geleitet. Der gebildete Didodecylether wurde anschließend im Vakuum destilliert.

**Tabelle 5**

| Dialkylether | Didodecyl-Ether | Ditetradecyl-Ether | Dihexadecyl-Ether | Dioctadecyl-Ether |
|---|---|---|---|---|
| Reinheit [%] | 93,4 | 95,2 | 94,8 | 91,2 |
| Onset [°C] | 30,4 | 41,8 | 51,5 | 59,3 |
| Schmelzwärme [J/g] | 209,4 | 227,4 | 231,2 | 207,9 |

## Patentansprüche

1. Verfahren zur Herstellung eines Latentwärmespeichermaterials umfassend den Schritt der Dehydratisierung von linearen Fettalkoholen zu Olefinen, wobei die Fettalkohole aus nativen pflanzlichen Rohstoffen gewonnen wurden und auf der Stufe der Alkohole durch Destillation aufgereinigt wurden, so dass
• größer 95 Massen% der eingesetzten Fettalkohole linear sind,
• größer 95 Massen% der eingesetzten Fettalkohole geradzahlige Kettenlängen aufweisen,
• größer 95 Massen% der eingesetzten Fettalkohole ausschließlich eine bestimmte C-Zahl aufweisen
und anschließende Hydrierung der Olefine zu Paraffinen.

2. Verfahren nach Anspruch 1, **dadurch gekennzeichnet, dass** die Paraffine zu größer 95 Massen%, insbesondere zu größer 98 Massen%, geradzahlige Kettenlängen aufweisen.

3. Verfahren nach Anspruch 1, **dadurch gekennzeichnet, dass** die Paraffine zu größer 97 Massen%, ausschließlich eine bestimmte C-Zahl aufweisen.

4. Verfahren nach zumindest einem der vorhergehenden Ansprüche, **dadurch gekennzeichnet, dass** die Fettalkohole Cetylalkohol oder Stearylalkohol sind.

5. Verfahren nach zumindest einem der vorhergehenden Ansprüche, **dadurch gekennzeichnet, dass** das Latentwärmespeichermaterial von einem Polymermaterial als Kapselwand zu Mikrokapseln mit mittleren Teilchengrößen im Bereich von 1 bis 200 µm oder zu Makrokapseln mit mittleren Teilchengrößen im Bereich von größer 200 µm bis 2 cm gekapselt wird.

6. Verfahren nach zumindest einem der Ansprüche 1 bis 5, **dadurch gekennzeichnet, dass** das Paraffin Hexadecan, Octadecan, Eicosan oder Docosan ist.

7. Verfahren nach zumindest einem der Ansprüche 1 oder 6, **dadurch gekennzeichnet, dass** die Fettalkohole zu größer 98 Massen% linear sind.

## Claims

1. Method for the production of a latent heat storage material, comprising the step of dehydrating of linear fatty alcohols to obtain olefins, in which the fatty alcohols are obtained from native vegetable raw materials and are purified by distillation when being alcohols so that:
• more than 95% by mass of the fatty alcohols used are linear,
• more than 95% by mass of the fatty alcohols used have even numbered chain lengths,
• more than 95% by mass of the fatty alcohols used exclusively have a certain C-number
and subsequent hydrogenation of the olefins to obtain paraffins.

2. Method according to claim 1, **characterized in that** the paraffins comprise even-numbered chain lengths at more than 95% by mass, in particular at more than 98% by mass.

3. Method according to claim 1, **characterized in that** the paraffins exclusively comprise a certain C-number at more than 97% by mass.

4. Method according to at least one of the preceding claims, **characterized in that** the fatty alcohols are cetyl alcohol or stearyl alcohol.

5. Method according to at least one of the preceding claims, **characterized in that** the latent heat storage material is encapsulated by a polymer material as the capsule wall material into microcapsules with average particle sizes in the range from 1 to 200 µm, or into macro-capsules with average particle sizes in the range from more than 200 µm to 2 cm.

6. Method according to at least one of the preceding claims 1 to 5, **characterized in that** the paraffin is hexadecane, octadecane, eicosane, or docosane.

7. Method according to at least one of the preceding claims 1 or 6, **characterized in that** the fatty alcohols are linear at more than 98 % by mass.

## Revendications

1. Procédé de fabrication d'un matériau à changement de phase comprenant l'étape de déshydrogénation d'alcools gras linéaires en oléfines, les alcools gras ayant été extraits de matières premières végétales natives et ayant été purifiés par distillation au stade d'alcools, de telle sorte que :
• plus de 95 % en poids des alcools gras mis en oeuvre sont linéaires,
• plus de 95 % en poids des alcools gras mis en oeuvre présentent des longueurs de chaîne en nombre pair,
• plus de 95 % en poids des alcools gras mis en oeuvre présentent exclusivement un nombre de C déterminé,
et l'hydrogénation consécutive des oléfines en paraffines.

2. Procédé selon la revendication 1, **caractérisé en ce que** les paraffines présentent à plus de 95 % en poids, en particulier à plus de 98 % en poids, des longueurs de chaîne en nombre pair.

3. Procédé selon la revendication 1, **caractérisé en ce que** les paraffines présentent à plus de 97 % en poids exclusivement un nombre de C déterminé.

4. Procédé selon au moins l'une des revendications précédentes, **caractérisé en ce que** les alcools gras sont l'alcool cétylique ou l'alcool stéarylique.

5. Procédé selon au moins l'une des revendications précédentes, **caractérisé en ce que** le matériau à changement de phase est encapsulé par un matériau polymère sous forme de paroi d'encapsulation pour donner des microcapsules ayant des tailles moyennes de particules dans la plage de 1 à 200 µm ou des macrocapsules ayant des tailles moyennes de particules dans la plage de plus de 200 µm à 2 cm.

6. Procédé selon au moins l'une des revendications 1 à 5, **caractérisé en ce que** la paraffine est l'hexadécane, l'octadécane, l'éicosane ou le docosane.

7. Procédé selon au moins l'une des revendications 1 ou 6, **caractérisé en ce que** les alcools gras sont linéaires à plus de 98 % en poids.
